# FASCICULE DE BREVET EUROPEEN

(11) **EP 1 039 297 B1**
(45) Date de publication et mention de la délivrance du brevet: **04.06.2003**
(21) Numéro de dépôt: 00400670.6
(22) Date de dépôt: 10.03.2000
(51) Int. Cl.: G01N 33/50

(54) **Réactif pour la détermination des leucocytes et la mesure de l'hémoglobine dans un échantillon de sang**
Reagenz zur Bestimmung von Leukozyten und zur Messung von Hämoglobin in einer Blutprobe
Reagent for the determination of leucocytes and the measurement of hemoglobin in a blood sample

(30) Priorité: 19.03.1999 FR 9903467
(43) Date de publication de la demande: 27.09.2000
(73) Titulaire: ABX, 34184 Montpellier Cedex 4 (FR)
(72) Inventeur: Veriac, Sylvie, Les Jardins d'Alhambra No.7, 34000 Montpellier (FR); Champseix, Henri, 34980 Montferrier sur Lez (FR)
(74) Mandataire: Bezault, Jean

(56) Documents cités:
- EP-A- 0 177 137
- EP-A- 0 442 776
- EP-A- 0 444 240
- EP-A- 0 695 936
- US-A- 4 286 963
- US-A- 4 617 275
- US-A- 4 983 375

## Description

L'invention se rapporte aux analyses hématologiques.

Elle concerne plus particulièrement un réactif pour la détermination des leucocytes totaux et la mesure de l'hémoglobine dans un échantillon de sang.

L'invention vise également à procurer un tel réactif qui permet l'identification d'une sous-population leucocytaire, en particulier les polynucléaires basophiles.

La détermination des leucocytes, en particulier de certaines sous-populations leucocytaires, ainsi que la mesure de la concentration en hémoglobine des érythrocytes ou globules rouges sont très importantes pour le diagnostic en pathologie humaine.

On rappelle ici que les leucocytes, ou globules blancs humains, sont divisés en cinq sous-populations, à savoir trois sous-populations principales : les lymphocytes, monocytes et polynucléaires ou granulocytes, ces derniers étant eux-mêmes subdivisés en neutrophiles, éosinophiles et basophiles, en fonction des caractéristiques de leurs granules cytoplasmiques.

La détermination des leucocytes totaux ainsi que l'identification de leurs différentes sous-populations sont réalisées par l'intermédiaire de techniques traditionnelles d'observation microscopique, ou de techniques plus modernes, fondées principalement sur la mesure de variations de résistivité (WO 84/03771) ou la diffraction optique (US 3 740 143) développées pour des appareils automatiques spécifiques.

Pour l'identification des sous-populations leucocytaires, la numération des granulocytes basophiles est particulièrement délicate étant donné que cette population ne représente, chez un individu sain, que 0,5% à 1 % de la population leucocytaire totale.

On observe une augmentation de cette population basophile, qui atteint alors une proportion de 2 à 3 % en poids, lors de réactions allergiques. Parmi les infections, ce sont la tuberculose et la varicelle qui peuvent entraîner une basophilie et, pour les maladies métaboliques, le myxoedème et les hyperlipidémies. Par conséquent, la numération des granulocytes basophiles revêt une importance particulière.

Le Brevet FR 90 01660 et son équivalent US 5 196 346 décrivent un réactif qui préserve les granulocytes basophiles, de façon à permettre leur détermination par mesure de résistivité. Cependant, ce réactif ne permet pas la mesure de l'hémoglobine.

On rappellera que l'hémoglobine est une chromo-protéine contenue dans les globules rouges du sang ou érythrocytes.

La mesure de la concentration en hémoglobine nécessite donc l'utilisation d'un réactif de lyse cellulaire capable de provoquer la lyse des érythrocytes, afin de libérer l'hémoglobine pour sa mesure.

Il est connu pour cela d'utiliser des réactifs contenant au moins un détergent et des ions cyanures capables de procéder à la transformation de l'hémoglobine en un composé chromogène pour permettre sa détermination par mesure de colorimétrie.

Un réactif cyanuré de ce type est décrit dans les Brevets US 3 874 852 et US 3 854 914.

Toutefois, ces réactifs ont pour inconvénient principal d'utiliser du cyanure. De plus, ils ne permettent pas d'identifier et de quantifier les sous-populations leucocytaires contenues dans l'échantillon de sang à analyser.

Il est à noter que des réactifs, ne contenant pas de cyanure et permettant la détermination des leucocytes en plus de la mesure de l'hémoglobine, ont déjà été proposés dans l'art antérieur.

Ainsi, le document WO 96 02841 décrit un réactif de mesure de l'hémoglobine sans cyanure, lequel contient un détergent ainsi qu'un sel d'hydroxylamine. Ce réactif peut être utilisé pour la numération des leucocytes totaux, mais aucune différentiation leucocytaire n'est possible.

Le Brevet US 5 242 832 décrit un réactif similaire qui permet également une identification leucocytaire partielle. Cependant, ce réactif ne permet pas l'identification des cellules basophiles, mais seulement l'évaluation des lymphocytes, monocytes et granulocytes.

Le document WO 98 32016 décrit aussi un réactif de ce type. Toutefois, les sous-populations granulocytaires minoritaires, que sont les éosinophiles et les basophiles, ne sont pas identifiées par le réactif décrit dans cette publication.

Le document US 4 286 963 décrit un réactif lytique unique pour la détermination différentielle de sous-populations de leucocytes (sans identification directe des polynucléaires basophiles) et de l'hémoglobine, comprenant un système tampon propre à ajuster le pH à une valeur acide, un détergent cationique et un composé sans cyanure de type phényl/phénoxy ou polyhydroxy. Ce réactif ne permet pas la détermination des polynucléaires basophiles.

Le document US 4 617 275 décrit aussi un réactif lytique unique analogue à celui du document US 4 286 963, dans lequel le composé sans cyanure est le 2-pyridylthio-1-oxyde. Ce réactif présente les mêmes inconvénients que celui du document US 4 286 963.

Le document EP 0 444 240 décrit un système de réactifs pour la classification des leucocytes, y compris les polynucléaires basophiles, comprenant un détergent non-ionique, un système tampon pour obtenir une valeur acide neutralisée avec un deuxième réactif et un agent solubilisant qui peut être une thiourée. Ce système de réactifs ne permet pas la mesure de l'hémoglobine.

Le document US 4 983 375 décrit un colorant hématologique comprenant une thiourée comme agent anti-oxydant.

L'invention a notamment pour but de surmonter les inconvénients des réactifs connus.

Elle vise en particulier à procurer un réactif d'analyse hématologique pour la détermination des leucocytes totaux, et en particulier l'identification et la quantification d'une sous-population leucocytaire, que constituent les cellules basophiles, et cela dans un échantillon de sang total.

L'invention vise également à procurer un réactif permettant notamment la lyse des érythrocytes ou globules rouges, nécessaire à la détermination des leucocytes ainsi qu'à la mesure de l'hémoglobine.

Elle vise aussi à procurer un tel réactif d'analyse hématologique sous la forme d'un réactif unique, et non pas d'un système de réactifs.

Elle vise encore à procurer un tel réactif d'analyse hématologique qui ne comporte pas de composés cyanurés.

En outre, l'invention vise à procurer un tel réactif hématologique convenant tout particulièrement aux automates hématologiques.

L'invention propose à cet effet un réactif d'analyse hématologique du type défini ci-dessus, lequel comprend essentiellement :
- un système tampon propre à ajuster sélectivement le pH du réactif à une valeur inférieure à 3 ;
- au moins un détergent de type cationique ; et
- un composé nitrogéné, qui est une thiourée.

Le système tampon du réactif est un constituant clé car le pH du réactif permet l'identification de la sous-population constituée par les cellules basophiles, lesquelles sont d'un intérêt particulier.

En effet, du fait de leurs caractéristiques biochimiques, les cellules basophiles sont susceptibles de résister plus longtemps que les autres sous-populations leucocytaires à l'agressivité d'un pH acide. Cette propriété permet donc leur isolement et leur identification, notamment par une mesure résistive.

Avantageusement, le système tampon est choisi pour que la valeur de pH du réactif soit égale à 2,4.

Le système tampon est avantageusement choisi parmi les suivants :
- chlorure de potassium / acide chlorhydrique ;
- acide tartarique / hydroxyde de sodium ;
- acide citrique / hydroxyde de sodium ;
- potassium hydrogénophtalate / acide chlorhydrique ;
- acide citrique / di-sodium hydrogénophosphate ; et
- acide borique / acide citrique / potassium dihydrogénophosphate.

Les détergents de type cationique exercent une fonction de lyse des globules rouges ou érythrocytes, ce qui permet de libérer l'hémoglobine qui peut être ensuite déterminée par mesure d'absorbance. D'une manière générale, les détergents cationiques, sont principalement utilisés pour dissocier les complexes protéiques et solubiliser les protéines des membranes. Ils sont dits dénaturants. Leur action est rapide et donc compatible avec les contraintes de cadence spécifiques aux appareils automatiques.

Le détergent est avantageusement choisi parmi les composés suivants :
- les amines primaires, les acétates et chlorhydrates d'amines grasses ;
- les sels d'ammonium quaternaire et le bromure de triméthylcéthyl ammonium ;
- les amides de diamines substituées, cationisées par le sulfate d'éthyle, la diéthanolamino-propylamine ou le diéthylamino-propylamide ; et
- les amides de diéthylénetriamine cyclisés.

Le composé nitrogéné à savoir la thiourée exerce essentiellement une fonction de stabilisation physico-chimique des dérivés d'oxydation de l'hémoglobine.

Ce composé nitrogéné est avantageusement la 1,3-diméthyl-2-thiourée.

Le réactif de l'invention peut comprendre en outre au moins un sel inorganique.

Ce sel, s'il est présent, intervient dans l'activité détergente et permet de maintenir dans les limites de la normalité les phénomènes d'osmose au niveau des membranes cellulaires, ce qui est important pour la détermination des cellules basophiles. Ce sel joue également un rôle dans les méthodes de mesure de résistivité qui sont généralement appliquées dans les automates hématologiques.

Le sel inorganique est avantageusement constitué par un sel de métal alcalin. En tant que sels utilisables, on peut citer principalement les chlorures ou sulfates de sodium ou de potassium.

De manière préférentielle, le détergent est présent à une concentration de 0,2-20 g/l et le composé nitrogéné (thiourée) à une concentration de 0,1-10 g/l.

L'invention sera décrite maintenant en référence à l'exemple non limitatif suivant.

### EXEMPLE

On prépare un réactif d'analyse hématologique à partir des composés ci-dessous, et dans les concentrations indiquées :

| Composés | Concentrations |
|---|---|
| chlorure de potassium | 5-10 g/l |
| 1,3-diméthyl-2-thiourée | 0,5-3 g/l |
| dodecyltriméthyl ammonium chlorure | 0,5-5 g/l |
| potassium hydrogénophosphate/HCl | 1,0-10 g/l |

Les composés ci-dessus sont mélangés et le pH est ajusté à une valeur acide inférieure à 3, et typiquement de l'ordre de 2,4.

A l'aide de ce réactif, on procède à des analyses hématologiques sur un échantillon de sang total humain en utilisant un automate d'hématologie.

Pour cela, on met en contact 10µl de l'échantillon de sang total avec 2ml du réactif ci-dessus à 35°C.

On procède à différents types d'analyses en comparant le réactif de l'invention avec un ou plusieurs réactifs de référence.

Le réactif de référence est une lyse utilisée dans las automates hématologiques afin de reproduire le dosage de l'hémoglobine suivant la méthodologie classique, non automatique, dite de Drabkin. Il s'agit du dosage de la cyanméthémoglobine.

Selon cette méthode, le fer ferreux (Fe⁺⁺) de l'hème des hémoglobine, oxyhémoglobine et carboxyhémoglobine contenues dans les globules rouges est oxydé en fer ferrique (Fe⁺⁺⁺) par le cyanure de fer pour former la méthémoglobine. La méthémoglobine se combine ensuite avec des ions cyanure pour former la cyanméthémoglobine, laquelle est mesurée par spectrophotométrie à 540 nm [**Drabkin**, J. Biol. Chem. 112:51 (1935)].

Par contre, le réactif de l'invention est utilisé dans un mode de dosage sans cyanure. L'hémoglobine érythrocytaire est éluée par l'action d'un agent de lyse approprié. Le fer hémique de l'hémoglobine éluée est oxydé par l'action combinée du composé érythrolytique et de l'oxygène dissous dans la solution. La méthémoglobine libre est instable si on la compare à la cyanméthémoglobine. On utilise donc des composés ayant des atomes donneurs d'électrons pour réduire le fer hémique et stabiliser la méthémoglobine.

Le réactif de l'invention est utilisé pour différents types d'analyses effectuées sur un automate hématologique.

### 1) Numération des leucocytes

On procède à une numération des leucocytes ou globules blancs totaux.

On compare les résultats de mesure obtenus avec le réactif de l'invention et avec un réactif de référence qui ne permet pas de différentiation leucocytaire même partielle.

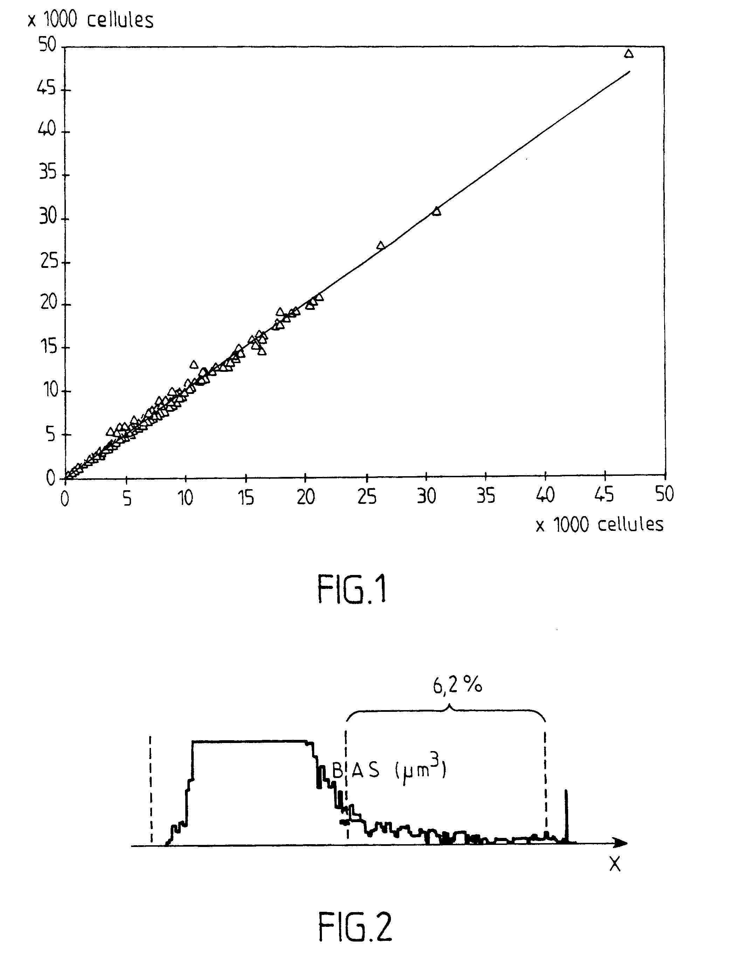

La figure 1 montre les valeurs de mesures exprimées en milliers de cellules, d'une part pour le réactif de référence représenté en abscisse, et d'autre part pour le réactif de l'invention représenté en ordonnée.

Le graphique montre une excellente corrélation entre les deux types de mesures. Les valeurs du coefficient de corrélation (R² = 0,99) et de la pente de la droite de régression (0,99) indiquent une très bonne corrélation.

### 2) Différentiation et numération des polynucléaires basophiles

La figure 2 montre la courbe résultant de l'analyse résistive d'un échantillon de sang total avec le réactif de l'invention décrit plus haut. Cette courbe représente un histogrammme de répartition des cellules en fonction de leurs tailles.

L'axe des x (abscisse) correspond à la détermination des volumes cellulaires (µm³) calculés par une mesure résistive. Les polynucléaires basophiles se situent à droite du curseur central (BAS). A gauche de ce curseur central se trouvent toutes les autres sous-populations leucocytaires non différentiables volumétriquement du fait de la forte agressivité du pH du réactif.

Dans l'exemple, on identifie des polynucléaires basophiles qui représentent une proportion de 6,2 % (en volume) par rapport à la population leucocytaire totale.

### 3) Mesure de l'hémoglobine

On effectue une mesure de l'hémoglobine avec le réactif de l'invention et on compare les résultats de mesures de la concentration en hémoglobine avec le réactif de l'invention et un réactif de référence contenant des composés cyanurés.

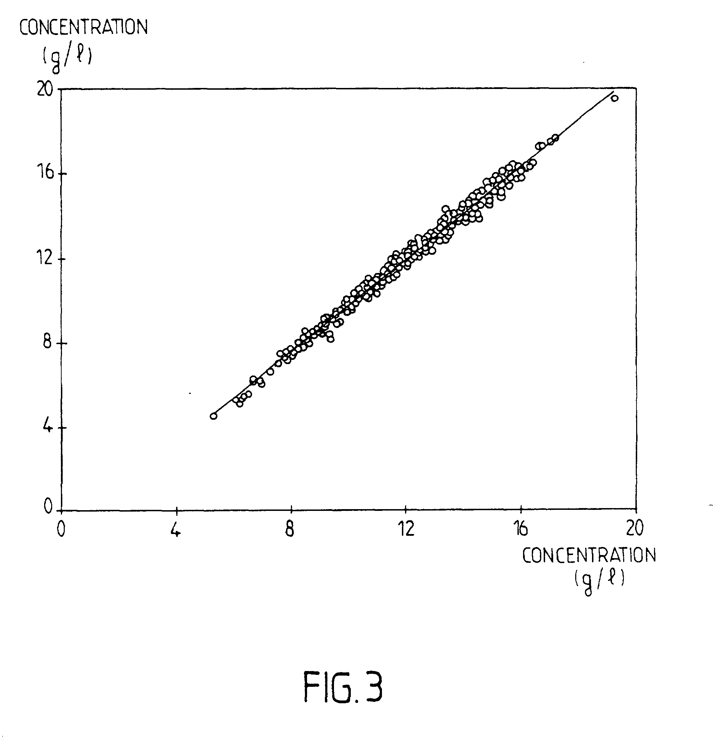

La figure 3 montre les valeurs de concentrations en hémoglobine (exprimées en grammes/litre) obtenues par le réactif de référence (représentation en abscisse) et le réactif de l'invention (représentation en ordonnée). Là encore, les valeurs du coefficient de corrélation (R2 = 0,99) et de la pente de la droite de régression (1,09) indiquent une très bonne corrélation.

Ainsi, l'invention procure un réactif unique qui permet la détermination des leucocytes totaux, l'identification d'une sous-population leucocytaire (en particulier les polynucléaires basophiles) et la mesure de l'hémoglobine, et cela sans utilisation de composés cyanurés.

Le réactif de l'invention présente notamment la particularité de permettre la mesure de l'hémoglobine dans des conditions très acides par rapport aux réactifs connus.

En outre, ce réactif se présente sous la forme d'un réactif unique, et non d'un système de plusieurs réactifs, et il convient tout spécialement aux analyses effectuées sur des automates hématologiques.

## Revendications

1. Réactif pour la détermination des leucocytes totaux et des polynucléaires basophiles, ainsi que pour la mesure de l'hémoglobine dans un échantillon de sang,
**caractérisé en ce qu'**il comprend :
- un système tampon propre à ajuster sélectivement le pH du réactif à une valeur inférieure à 3 ;
- au moins un détergent de type cationique ;
- un composé nitrogéné, qui est une thiourée.

2. Réactif selon la revendication 1, **caractérisé en ce que** le système tampon est propre à ajuster le pH à une valeur égale à 2,4.

3. Réactif selon la revendication 1 ou 2, **caractérisé en ce que** le système tampon est choisi parmi :
- chlorure de potassium / acide chlorhydrique ;
- acide tartarique / hydroxyde de sodium ;
- acide citrique / hydroxyde de sodium ;
- potassium hydrogénophtalate / acide chlorhydrique ;
- acide citrique / di-sodium hydrogénophosphate ; et
- acide borique / acide citrique / potassium dihydrogénophosphate.

4. Réactif selon l'une des revendications 1 à 3, **caractérisé en ce que** le détergent est choisi parmi :
- les amines primaires, les acétates et chlorhydrates d'amines grasses ;
- les sels d'ammonium quaternaire et le bromure de triméthylcéthyl ammonium ;
- les amides de diamines substituées, cationisées par le sulfate d'éthyle, la diéthanolamino-propylamine ou le diéthylamino-propylamide ; et
- les amides de diéthylénetriamine cyclisés.

5. Réactif selon l'une des revendications 1 à 4, **caractérisé en ce que** le composé nitrogéné est la 1,3-diméthyl-2-thiourée.

6. Réactif selon l'une des revendications 1 à 5, **caractérisé en ce qu'**il comprend en outre au moins un sel inorganique.

7. Réactif selon la revendication 6, **caractérisé en ce que** le sel inorganique est un sel de métal alcalin.

8. Réactif selon la revendication 6 ou 7, **caractérisé en ce que** le sel inorganique est choisi parmi les chlorures et sulfates de sodium ou de potassium.

9. Réactif selon l'une des revendications 1 à 8, **caractérisé en ce que** le détergent est présent à une concentration de 0,2-20 g/l et le composé nitrogéné à une concentration de 0,1-10 g/l.

10. Réactif selon l'une des revendications 1 à 9, **caractérisé par** la composition suivante :
| Composés | Concentrations |
|---|---|
| chlorure de potassium | 5-10 g/l |
| 1,3-diméthyl-2-thiourée | 0,5-3 g/l |
| dodecyltriméthyl ammonium chlorure | 0,5-5 g/l |
| potassium hydrogénophosphate/HCl | 1,0-10 g/l |

## Patentansprüche

1. Reagenz für die Bestimmung der Gesamtheit von Leukozyten und von mehrkernigen Basophilen, sowie für die Messung von Hämoglobin in einer Blutprobe,
**dadurch gekennzeichnet,**
**daß** es folgendes aufweist:
- ein Puffersystem, das geeignet ist, den pH-Wert des Reagenz selektiv auf einen Wert unterhalb von 3 einzustellen;
- mindestens ein kationisches Detergenz;
- eine Stickstoffverbindung, die ein Thioharnstoff ist.

2. Reagenz nach Anspruch 1,
**dadurch gekennzeichnet,**
**daß** das Puffersystem geeignet ist, den pH-Wert auf einen Wert einzustellen, der gleich 2,4 ist.

3. Reagenz nach einem der Ansprüche 1 oder 2,
**dadurch gekennzeichnet,**
**daß** das Puffersystem aus den folgenden ausgewählt ist:
- Kaliumchlorid / Salzsäure;
- Weinsäure / Natriumhydroxid;
- Zitronensäure / Natriumhydroxid;
- Kaliumhydrogenphtalat / Salzsäure;
- Zitronensäure / Dinatriumhydrogenphosphat; und
- Borsäure / Zitronensäure / Kaliumdihydrogenphosphat.

4. Reagenz nach einem der Ansprüche 1 bis 3,
**dadurch gekennzeichnet,**
**daß** das Detergenz aus den folgenden ausgewählt ist:
- primären Aminen, Acetaten und Chlorhydraten von Fettaminen;
- quartären Ammoniumsalzen und dem Bromid von Trimetylcethylammonium;
- Amiden von substituierten Diaminen, die mittels Ethylsulfat, Diethanolaminopropylamin oder Diethylaminopropylamid kationisiert sind; und
- Amiden von zyklischem Diethylentriamin.

5. Reagenz nach einem der Ansprüche 1 bis 4,
**dadurch gekennzeichnet,**
**daß** die Stickstoffverbindung 1, 3-Dimethyl-2-Thioharnstoff ist.

6. Reagenz nach einem der Ansprüche 1 bis 5,
**dadurch gekennzeichnet,**
**daß** es ferner mindestens ein anorganisches Salz aufweist.

7. Reagenz nach Anspruch 6,
**dadurch gekennzeichnet,**
**daß** das anorganische Salz ein Salz eines Alkalimetalls ist.

8. Reagenz nach einem der Ansprüche 6 oder 7,
**dadurch gekennzeichnet,**
**daß** das anorganische Salz ausgewählt ist aus den Chloriden und Sulfaten von Natrium oder Kalium.

9. Reagenz nach einem der Ansprüche 1 bis 8,
**dadurch gekennzeichnet,**
**daß** das Detergenz in einer Konzentration von 0,2 g/l bis 20 g/l und die Stickstoffverbindung in einer Konzentration von 0,1 g/l bis 10 g/l vorhanden ist.

10. Reagenz nach einem der Ansprüche 1 bis 9,
**gekennzeichnet durch**
die folgende Zusammensetzung:
| Bestandteile | Konzentrationen |
|---|---|
| Kaliumchlorid | 5 g/l - 10 g/l |
| 1, 3-Dimethyl-2-Thioharnstoff | 0,5 g/l - 3 g/l |
| Dodecyltrimethylammoniumchlorid | 0,5 g/l - 5 g/l |
| Kaliumhydrogenphosphat / Salzsäure | 1,0 g/l - 10 g/l |

## Claims

1. Reagent for determining total leukocytes and basophil polynuclear cells and also for measuring haemoglobin in.a blood sample,
**characterized in that** it comprises:
- a buffer system able to selectively adjust the pH of the reagent to a value of less than 3;
- at least one detergent of the cationic type;
- a nitrogenous compound, which is a thiourea.

2. Reagent according to Claim 1, **characterized in that** the buffer system is able to adjust the pH to a value equal to 2.4.

3. Reagent according to Claim 1 or 2, **characterized in that** the buffer system is chosen from:
- potassium chloride / hydrochloric acid;
- tartaric acid / sodium hydroxide;
- citric acid / sodium hydroxide;
- potassium hydrogen phthalate / hydrochloric acid;
- citric acid / disodium hydrogen phosphate; and
- boric acid / citric acid / potassium dihydrogen phosphate.

4. Reagent according to one of Claims 1 to 3, **characterized in that** the detergent is chosen from:
- primary amines, acetates and hydrochlorides of fatty amines;
- quaternary ammonium salts and trimethylcetylammonium bromide;
- amides of substituted diamines, cationized with ethyl sulphate, diethanolaminopropylamine or diethylaminopropylamide; and
- cyclized diethylenetriamine amides.

5. Reagent according to one of Claims 1 to 4, **characterized in that** the nitrogenous compound is 1,3-dimethyl-2-thiourea.

6. Reagent according to one of Claims 1 to 5, **characterized in that** it also comprises at least one inorganic salt.

7. Reagent according to Claim 6, **characterized in that** the inorganic salt is an alkali metal salt.

8. Reagent according to Claim 6 or 7, **characterized in that** the inorganic salt is chosen - from sodium chloride or sulphate and potassium chloride or sulphate.

9. Reagent according to one of Claims 1 to 8, **characterized in that** the detergent is present at a concentration of 0.2-20 g/l and the nitrogenous compound is present at a concentration of 0.1-10 g/l.

10. Reagent according to one of Claims 1 to 9, **characterized by** the following composition:
| Compounds | Concentrations |
|---|---|
| potassium chloride | 5-10 g/l |
| 1,3-dimethyl-2-thiourea | 0.5-3 g/l |
| dodecyltrimethylammonium chloride | 0.5-5 g/l |
| potassium hydrogen phosphate/HCl | 1.0-10 g/l |
